# EUROPÄISCHE PATENTSCHRIFT

(11) **EP 0 832 687 B1**
(45) Veröffentlichungstag und Bekanntmachung des Hinweises auf die Patenterteilung: **31.10.2001**
(21) Anmeldenummer: 97111737.9
(22) Anmeldetag: 10.07.1997
(51) Int. Cl.: C07C 319/08, B01J 23/04, B01J 23/30

(54) **Katalysator, Verfahren zu seiner Herstellung und Verwendung zur Synthese von Methylmercaptan**
Catalyst, process for its production and use in synthesis of methylmercaptan
Catalyseur, son procédé de préparation et son utilisation pour la synthèse de méthylmercaptan

(30) Priorität: 26.09.1996 DE 19639584
(43) Veröffentlichungstag der Anmeldung: 01.04.1998
(73) Patentinhaber: Degussa AG, 40474 Düsseldorf (DE)
(72) Erfinder: Sauer, Jörg, Dr., 63517 Rodenbach (DE); Böck, Wolfgang, Dr., 63505 Langenselbold (DE); von Hippel, Lukas, Dr., 63755 Alzenau (DE); Burkhardt, Werner, 63636 Brachttal (DE); Rautenberg, Stephen, Dr., 63457 Hanau (DE); Arntz, Dietrich, Dr., 63829 Oberursel (DE); Hofen, Willi, 63517 Rodenbach (DE)

(56) Entgegenhaltungen:
- US-A- 2 820 062
- A. V. MASHKINA ET AL.: "Activity of tungstate catalysts in the synthesis of methylcaptane from methanol and hydrogen sulfide" REACT. KINET. CATAL. LETT., Bd. 36, Nr. 1, 1988, Seiten 159-164, XP002063476

## Beschreibung

Die vorliegende Erfindung betrifft einen Katalysator zur Synthese von Methylmercaptan aus Methanol und Schwefelwasserstoff, sowie ein Verfahren zur Herstellung dieses Katalysators.

Methylmercaptan ist ein industriell wichtiges Zwischenprodukt für die Synthese von Methionin sowie für die Herstellung von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drucken zwischen 1 und 25 bar.

Das Reaktionsgasgemisch enthält neben dem gebildeten Methylmercaptan die nicht umgesetzten Ausgangsstoffe und Nebenprodukte, wie zum Beispiel Dimethylsulfid und Dimethyläther sowie die im Sinne der Reaktion inerten Gase, wie zum Beispiel Methan, Kohlenmonoxid, Wasserstoff und Stickstoff. Aus diesem Reaktionsgemisch wird das gebildete Methylmercaptan abgetrennt.

Wird die Reaktion von Schwefelwasserstoff und Methanol am Katalysator bei erhöhtem Druck durchgeführt und fällt somit das Produkt der Methylmercaptan-Herstellung bei erhöhtem Druck (mehr als 7 bar) an, kann, wie in DE 17 68 826 beschrieben ist, Methylmercaptan beispielsweise durch Wäsche mit Methanol bei einer Temperatur am Kopf des Wäschers von 25°C abgetrennt werden. Fällt das Reaktionsprodukt bei Normaldruck an, muß in der Aufarbeitung bei Temperaturen bis -60°C gearbeitet werden (JP-OS 45-10728), um das Methylmercaptan in flüssiger Form gewinnen zu können. Der nicht umgesetzte Schwefelwasserstoff kann, wie in DE 17 68 826 beschrieben, wieder zum Reaktor zurückgeführt werden.

Für die Wirtschaftlichkeit des Verfahrens wird eine möglichst hohe Selektivität bei der katalytischen Reaktion von Methanol und Schwefelwasserstoff zu Methylmercaptan gefordert, um den Aufwand bei der Abtrennung des gebildeten Methylmercaptans vom Reaktionsgasgemisch so gering wie möglich zu halten. Hier stellt insbesondere der Energieaufwand zur Kühlung des Reaktionsgasgemisches zur Kondensation des Methylmercaptans einen großen Kostenfaktor dar.

Zur Erhöhung von Aktivität und Selektivität des Aluminiumoxidkatalysators wird dieser üblicherweise mit Kaliumwolframat promotiert. Dabei wird der Promotor gewöhnlich in Mengen bis 15 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet ällerdings auch einen hohen Überschuß des Schwefelwasserstoffes im Reaktionsgasgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen. Weiterhin ist es zur Verminderung der Wärmeverluste am Reaktor wünschenswert, die Reaktion bei möglichst geringen Temperaturen durchzuführen.

Die US-PS 2,820,062 beschreibt ein Verfahren zur Herstellung von organischen Thiolen, welches einen Katalysator aus aktivem Aluminiumoxid verwendet, der mit Kaliumwolframat in einer Menge von 1,5 bis 15 Gew.-%, bezogen auf das Gewicht des Katalysators, promotiert ist. Mit diesem Katalysator werden gute Aktivitäten und Selektivitäten bei Reaktionstemperaturen von 400°C und Molverhältnissen von 2 erzielt. Diese US-Patentschrift nennt verschiedene Möglichkeiten zur Einbringung des Kaliumwolframats in das Aluminiumoxid. So sollen Imprägnierverfahren, Copräzipitationen und reine Mischungen anwendbar sein. Der eigentlichen Herstellung des Katalysators wird wenig Bedeutung für die Wirtschaftlichkeit des Syntheseverfahrens von Methylmercaptan eingeräumt.

Aufgabe der vorliegenden Erfindung ist es, einen Katalysator und ein Verfahren zu seiner Herstellung anzugeben, welcher sich bei niedrigen Molverhältnissen von Schwefelwasserstoff zu Methanol durch verbesserte Aktivität und Selektivität gegenüber den bekannten Katalysatoren auszeichnet und somit zu einer besseren Wirtschaftlichkeit des Verfahrens führt.

Diese Aufgabe wird durch einen Katalysator aus einem Formkörper von Aluminiumoxid gelöst, welcher ein Alkaliwolframat als Promotor enthält. Der Katalysator ist dadurch gekennzeichnet, daß es sich bei dem Promotor um Cäsiumwolframat handelt, welches in einer Menge von mehr als 15 bis 40, bevorzugt 20 bis 35 Gew.-%, bezogen auf das Gesamtgewicht des fertigen Katalysators, aufgebracht ist.

Es wurde gefunden, daß Cäsiumwolframat, im Vergleich zu dem im Stand der Technik ausschließlich verwendeten Kaliumwolframat, dem Katalysator eine verbesserte Aktivität bei gleichzeitig verbesserter Selektivität verleiht. Diese verbesserten Leistungsdaten werden erst bei wesentlich höheren Konzentrationen des erfindungsgemäß zu verwendenden Promotors als beim konventionellen Promotor Kaliumwolframat erreicht. Unterhalb einer Konzentration von 15 Gew.-% weist Cäsiumwolframat keine Vorteile gegenüber Kaliumwolframat auf. Während Kaliumwolframat mit weiter steigender Konzentration keine weiteren Verbesserungen der Leistungsdaten des Katalysators erbringt, zeigt sich bei Verwendung von Cäsiumwolframat unerwarteter Weise eine Verbesserung von Aktivität und Selektivität bis über eine Konzentration von 25 Gew.-% hinaus. Bei Überschreiten der oberen Konzentrationsgrenze von 40 Gew.-% werden keine wesentlichen Leistungssteigerungen mehr erzielt.

Als Aluminiumoxid für diesen Katalysator wird sogenanntes aktives Aluminiumoxid eingesetzt. Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Enzyclopedia of Industrial Chemistry von 1985, Vol. A1, Seiten 561 - 562). Zu diesen Übergangsoxiden gehören χ-, κ-, γ-, δ-, η- und θ-Aluminiumoxid. Alle diese kristallographischen Phasen gehen bei Erhitzung des Aluminiumoxids auf Temperaturen über 1100°C in das thermisch stabile α-Aluminiumoxid über.

Aktives Aluminiumoxid wird kommerziell für katalytische Anwendungen in verschiedenen Qualitäten und Lieferformen angeboten. Gut geeignet für die Zwecke der Erfindung sind Formkörper in Form von granuliertem oder stranggepreßtem Aluminiumoxid mit Korndurchmessern von 1 bis 5 mm, einer spezifischen Oberfläche von 180 - 400 m²/g, einem Gesamtporenvolumen zwischen 0,3 und 1,0 ml/g sowie einer Schüttdichte von 300 bis 900 g/l. Für die Zwecke der Erfindung wird bevorzugt Aluminiumoxid mit mehr als 200 m²/g spezifischer Oberfläche verwendet, da die katalytische Aktivität des fertigen Katalysators mit steigender Oberfläche des Aluminiumoxids leicht ansteigt.

Die.wäßrige Imprägnierlösung von Cäsiumwolframat kann in einfacher Weise aus käuflicher Wolframsäure (H₂WO₄) und Cäsiumhydroxid (CsOH.H₂O) hergestellt werden. Hierzu wird Wolframsäure in Wasser suspendiert und unter Zugabe einer Base und Erwärmen aufgelöst. Cäsiumhydroxid wird getrennt von Wolframsäure ebenfalls in Wasser gelöst. Beide Lösungen werden anschließend vereinigt. Zur Stabilisierung der fertigen Imprägnierlösung sollte ihr pH-Wert im alkalischen Bereich zwischen 8 und 14 liegen. Ohne diese Maßnahme kann keine klare Lösung erhalten werden.

Zur Stabilisierung der Imprägnierlösung eignen sich anorganische und auch organische Basen. Bevorzugt werden solche Basen eingesetzt, die sich durch eine abschließende Wärmebehandlung des Katalysators rückstandslos austreiben lassen. Zu diesen Basen gehören Ammoniumhydroxid und organische Basen wie verschiedene Amine.

Vor dem Aufbringen der Imprägnierlösung ist es zweckmäßig, die Formkörper aus Aluminiumoxid durch Calcinieren bei Temperaturen zwischen 250 und 600°C für die Dauer von 1 bis 10, bevorzugt 2 bis 6 Stunden, von adsorbiertem Wasser und anderen flüchtigen Verunreinigungen zu befreien. Diese Vorbehandlung ist jedoch nicht zwingend notwendig.

Für das Aufbringen des Cäsiumwolframats können verschiedene Imprägniertechniken wie das Tauchimprägnieren, Sprühimprägnieren und die Porenvolumenimprägnierung in einem oder mehreren Schritten eingesetzt werden. Das gewählte Imprägnierverfahren muß es nur ermöglichen, die gewünschte hohe Beladungsmenge an Cäsiumwolframat auf dem Aluminiumoxid mit guter Gleichmäßigkeit über den gesamten Querschnitt der Formkörper aufzubringen. Die Gleichmäßigkeit der Imprägnierung kann zusätzlich durch eine langsame Vortrocknung des imprägnierten Aluminiumoxids über mehrere Stunden bei Raumtemperatur verbessert werden.

Bevorzugt wird Cäsiumwolframat durch Porenvolumenimprägnierung in einem einzigen Schritt auf die Formkörper aufgebracht. Nach Vortrocknung für die Dauer von 2 bis 20 Stunden bei Raumtemperatur hat sich das anfängliche Konzentrationsgefälle über den Querschnitt der Formkörper weitgehend ausgeglichen. Anschließend werden die so erhaltenen Katalysatorvorstufen für die Dauer von 1 bis 10 Stunden bei 300 bis 600°C calciniert. Hierdurch wird das Cäsiumwolframat auf dem Aluminiumoxid fixiert und die Base der Imprägnierlösung zersetzt und ausgetrieben.

Um eine Schädigung des Katalysators durch ein zu schnelles Austreiben der restlichen Feuchte zu vermeiden, sollte die Temperatur nach der Vortrocknung langsam von Raumtemperatur auf die Endtemperatur für die Calcinierung erhöht werden. Alternativ kann auch eine separate Zwischentrocknung bei 100 bis 200°C für die Dauer von etwa 0,5 bis 4 Stunden angewendet werden.

Für die folgenden Beispiele wurden zwei verschiedene Sorten Aluminiumoxid eingesetzt. Ihre Eigenschaften sind in der folgenden Tabelle 1 aufgeführt.

**Tabelle 1**

| Eigenschaften von Aluminiumoxid | | |
|---|---|---|
| | Aluminiumoxid I | Aluminiumoxid II |
| Hersteller | Norton | Rhône-Poulenc |
| Typ | SA 6276 | Spheralite 501a |
| spez. Oberfläche | 200 m²/g | 320 m²/g |
| Schüttdichte | 420-590 g/l | 800 g/l |
| Porenvolumen | 0,9-1 ml/g | 0,45 ml/g |
| Wasseraufnahme | 90g/100g Al₂O₃ | 52g/100g Al₂O₃ |
| Granulat ⌀ | 3 mm | 3 mm |

### Vergleichsbeispiel 1:

1 Kg Aluminiumoxid I wurde mit 8 Gew.-% Kaliumwolframat mit Hilfe der Porenvolumenimprägnierung imprägniert. Hierzu wurde im einzelnen wie folgt vorgegangen:

Das Granulat wurde zunächst für die Dauer von 4 Stunden bei 455°C an Luft calciniert.

Zur Herstellung der Kaliumwolframatlösung wurden 66,6 g Wolframsäure in 500 ml Wasser suspendiert und 29,9 g Kaliumhydroxid in 450 ml Wasser gelöst. Beide Komponenten wurden anschließend zusammengegossen und unter Rühren auf 95°C erwärmt, wobei sich eine klare Lösung bildete, die über das in einem Dragierkessel umgewälzte Granulat versprüht wurde. Das Volumen der Imprägnierlösung entsprach etwa 110% der experimentell bestimmten Wasseraufnahme der vorgelegten Granulatmenge.

Das imprägnierte Granulat wurde für die Dauer von 16 Stunden an Luft gelagert und anschließend bei 160°C für die Dauer von 2 Stunden zur Entfernung der Restfeuchte getrocknet. Danach wurde das Granulat 4 Stunden lang bei 455°C an Luft calciniert.

### Vergleichsbeispiel 2:

Vergleichsbeispiel 1 wurde wiederholt, jedoch wurde das Granulat mit 16 Gew.-% Kaliumwolframat belegt. Hierzu wurden die Einsatzmengen an Wolframsäure und Kaliumhydroxid entsprechend vergrößert.

### Vergleichsbeispiel 3:

1 Kg Aluminiumoxid II wurde analog zu Vergleichsbeispiel 1 mit 8 Gew.-% Kaliumwolframat bezogen auf das Gesamtgewicht des imprägnierten Aluminiumoxids belegt.

Hierzu wurden 66,6 g Wolframsäure in 400 ml Wasser suspendiert, 29,9 g Kaliumhydroxid in 170 ml Wasser gelöst und anschließend beide Komponenten unter Erwärmung auf 95°C miteinander verrührt, wobei sich eine klare Lösung bildete. Wegen des geringeren Porenvolumens von Aluminiumoxid II entsprach das Volumen dieser Imprägnierlösung etwa 110% der experimentell bestimmten Wasseraufnahme der vorgelegten Granulatmenge.

### Vergleichsbeispiel 4:

Vergleichsbeispiel 3 wurde wiederholt, jedoch wurde das Granulat mit 16 Gew.-% Kaliumwolframat belegt. Hierzu wurden die Einsatzmengen an Wolframsäure und Kaliumhydroxid entsprechend vergrößert.

### Vergleichsbeispiel 5:

Vergleichsbeispiel 4 wurde mit einer auf 20 Gew.-% erhöhten Beladungsmenge an Kaliumwolframat wiederholt.

### Vergleichsbeispiel 6:

Aluminiumoxid II wurde in einer zweistufigen Imprägnierung mit insgesamt 16 Gew.-% Kaliumwolframat belegt. In der ersten Imprägnierstufe wurde mit einem Überschuß an Imprägnierlösung gearbeitet. In der zweiten Imprägnierstufe wurde die Porenvolumenimprägnierung angewendet.

Im einzelnen wurde wie folgt vorgegangen: 1 Kg Aluminiumoxid II wurden 4 Stunden lang bei 455°C an Luft calciniert. Über das in einem Gefäß befindliche Granulat wurde eine vorbereitete Lösung von 8,7 Gew.-% Kaliumwolframat in Wasser mit einer Temperatur von 95°C gegossen, bis alle Katalysatorpartikel bedeckt waren. Nach einer Wartezeit von 40 Minuten wurde das überschüssige Wasser abgegossen, die feuchten Katalysatorteilchen 16 Stunden an Luft bei Raumtemperatur vorgetrocknet und anschließend 2 Stunden bei 120°C getrocknet. Auf den Katalysatorpartikeln hatten sich durch diese Behandlung 7 Gew.-% Kaliumwolframat, d.h. 75 g, abgelagert.

Zur Durchführung der Porenvolumenimprägnierung wurden 115,2 g Kaliumwolframat in 520 ml Wasser, entsprechend 100 % des gemessenen Wasseraufnahmevermögens des Katalysatormaterials, bei einer Temperatur von 95°C gelöst und über das in einem Dragierkessel umgewälzte Granulat verteilt. Daran schloß sich wieder eine 16-stündige Vortrocknung an Luft an, gefolgt von einer zweistündigen Trocknung bei 110°C. Abschließend wurden die Katalysatorteilchen 4 Stunden bei 455°C an Luft calciniert.

### Vergleichsbeispiel 7:

1 Kg Aluminiumoxid II wurde mit 8 Gew.-% Cäsiumwolframat analog der Vorgehensweise von Vergleichsbeispiel 3 imprägniert.

Hierzu wurden 42,3 g Wolframsäure in 400 ml Wasser suspendiert und 56,9 g CsOH.H₂O in 170 ml Wasser gelöst. Nach Vermischen beider Lösungen wurde unter Rühren auf 65°C erwärmt, wobei sich eine leicht trübe Lösung bildete. Eine Erwärmung der Lösung auf 95°C wie im Falle von Kaliumwolframat war nicht möglich, da die Lösung bei höheren Temperaturen einen Niederschlag bildet.

Imprägnierung, Lagerung und Calcinierung wurden wie schon in den beiden vorangegangenen Vergleichsbeispielen vorgenommen.

### Beispiel 1:

1 Kg Aluminiumoxid II wurde mit 20 Gew.-% Cäsiumwolframat belegt. Hierzu wurden 121,6 g Wolframsäure in 140 ml Wasser suspendiert und durch Zugabe von 260 ml 25%-tiger Ammoniaklösung und Erwärmen auf 50°C vollständig gelöst. 163,5 g CsOH.H₂O wurden in 170 ml Wasser gelöst und mit der ersten Lösung vermischt.

Vorcalcinieren, Imprägnieren, Trocknen und abschließendes Calcinieren wurden wie in den vorangegangenen Beispielen durchgeführt.

### Beispiel 2 und 3:

Beispiel 1 wurde mit 25%-tiger und 30%-tiger Beladung des Aluminiumoxids mit Cäsiumwolframat wiederholt.

### Anwendungsbeispiel

Die Katalysatoren wurden bezüglich ihrer Leistungsdaten bei der Synthese von Methylmercaptan aus Schwefelwasserstoff und Methanol getestet.

Die Synthese wurde in einem Edelstahlrohr von 14 mm Innendurchmesser und einer Länge von 500 mm durchgeführt. Die Katalysatorschüttung von jeweils 32,4 ml wurde beiderseits durch Inertschüttungen aus Glaskugeln im Reaktionsrohr fixiert. Das Reaktionsrohr wurde elektrisch auf die Reaktionstemperatur von etwa 350°C aufgeheizt.

Die Produkte Methylmercaptan, Dimethylsulfid, Dimethylether und nicht umgesetztes Methanol wurden nach Abkühlen des Produktes mit Methanol bei 25°C aus dem Gasstrom ausgewaschen und destillativ aufgearbeitet.

Die Versuchsbedingungen sind der folgenden Aufstellung zu entnehmen.

| | |
|---|---|
| GHSV | 1280 h⁻¹ (bezogen auf Normbedingungen) |
| LHSV | 0,56 h⁻¹ (bezogen auf flüssiges MeOH) |
| Reaktionstemperatur | 300-400°C |
| Molverh. H₂S/MeOH | 1,5 |
| Druck | 9 bar |

Die am Reaktionsgasgemisch durch on-line Gaschromatographie gewonnenen Meßergebnisse sowie die Zusammensetzung der geprüften Katalysatoren sind der Tabelle 2 zu entnehmen. Als Maß für Aktivität der Katalysatoren wurde die für einen Methanolumsatz von 90% erforderliche Reaktionstemperatur T_{90%} bestimmt. Tabelle 2 enthält außerdem die in diesem Arbeitspunkt ermittelte Selektivität.

Wie die Ergebnisse der Tabelle 2 zeigen, verbessert die Verwendung eines Aluminiumoxids mit höherer spezifischer Oberfläche die Aktivität des Katalysators und auch in geringerem Umfange seine Selektivität. Die Steigerung der Kaliumwolframatbeladung von 8 auf 16 Gew.-% verschlechtert zwar die Aktivität des Katalysators, verbessert jedoch seine Selektivität ganz entscheidend. Eine weitere Steigerung der Beladung auf 20 Gew.-% verschlechtert die Aktivität weiter. Die Selektivität nimmt nur noch im geringeren Maße zu, so daß Katalysatoren mit mehr als 16 Gew.-% Kaliumwolframat keine gesteigerte Wirtschaftlichkeit bei der Methylmercaptanherstellung ermöglichen.

Ein ähnliches Verhalten war auch für die Beladung mit Cäsiumwolframat zu erwarten. Überraschenderweise zeigen jedoch mit Cäsiumwolframat promotierte Katalysatoren eine wesentlich gesteigerte Aktivität bei gleichzeitig guter Selektivität. Die Selektivität kann durch Erhöhung der Beladung auf über 20 Gew.-% bis auf 92% gesteigert werden, ohne daß dadurch die Aktivität unverhältnismäßig verschlechtert wird.

**Tabelle 2**

| Versuchsergebnisse für Methanolumsatz von 90% | | | | | |
|---|---|---|---|---|---|
| **Katalysator** | **Aluminium oxid** | **Promotor** | **Beladung [Gew.-%]** | **T**_{**90%**} **[°C]** | **Selektivitä**t **[%]** |
| VB1 | I | K₂WO₄ | 8 | 360 | 79,8 |
| VB2 | I | K₂WO₄ | 16 | 382 | 86,8 |
| VB3 | II | K₂WO₄ | 8 | 347 | 79,9 |
| VB4 | II | K₂WO₄ | 16 | 357 | 89,2 |
| VB5 | II | K₂WO₄ | 20 | 365 | 91,5 |
| VB6^{*)} | II | K₂WO₄ | 16 | 357 | 91,3 |
| VB7 | II | Cs₂WO₄ | 8 | 323 | 73,6 |
| B1 | II | Cs₂WO₄ | 20 | 345 | 87,2 |
| B2 | II | Cs₂WO₄ | 25 | 349 | 92,0 |
| B3 | II | Cs₂WO₄ | 32,5 | 353 | 92,2 |
| VB1: Katalysator nach Vergleichsbeispiel 1 | | | | | |

| | | | | | |
|---|---|---|---|---|---|
| ^{*)} zweistufige Imprägnierung | | | | | |

Die vorstehenden Ausführungen wurden der Einfachheit halber auf die Probleme bei der Synthese von Methylmercaptan beschränkt. Dem Fachmann ist jedoch klar, daß der erfindungsgemäße Katalysator ebenso für die Synthese allgemeiner Mercaptane durch katalytische Umsetzung von olefinischen Kohlenwasserstoffen mit Schwefelwasserstoff geeignet ist.

## Patentansprüche

1. Katalysator aus einem Formkörper von Aluminiumoxid für die Synthese von Methylmercaptan, welcher ein Alkaliwolframat als Promotor enthält,
**dadurch gekennzeichnet,**
**daß** es sich bei dem Promotor um Cäsiumwolframat handelt, welches in einer Menge von mehr als 15 bis 40 Gew.-% bezogen auf das Gesamtgewicht des fertigen Katalysators aufgebracht ist.

2. Katalysator nach Anspruch 1,
**dadurch gekennzeichnet,**
**daß** das Aluminiumoxid eine spezifischen Oberfläche zwischen 180 und 400 m²/g aufweist.

3. Verfahren zur Herstellung des Katalysators nach Anspruch 1 mit den folgenden Verfahrensschritten
a) Imprägnieren der Formkörper aus Aluminiumoxid mit einer wäßrigen, alkalischen Lösung von Cäsiumwolframat,
b) Trocknen der erhaltenen Katalysatorvorstufen bei Raumtemperatur über mehrere Stunden und
c) abschließendes Calcinieren für die Dauer von 2 bis 10 Stunden bei Temperaturen von 300 bis 500°C.

4. Verwendung des Katalysators nach den Ansprüchen 1 bis 2 für die Synthese von Alkylmercaptanen aus Alkanolen und Schwefelwasserstoff.

5. Verwendung des Katalysators nach den Ansprüchen 1 bis 2 für die Synthese von Methylmercaptan aus Methanol und Schwefelwasserstoff.

## Claims

1. Catalyst comprising a moulding of aluminium oxide for the synthesis of methyl mercaptan, said catalyst containing an alkali metal tungstate as promoter,
**characterised in that**
the promoter is caesium tungstate, which is applied in a quantity of greater than 15 to 40 wt.%, relative to the total weight of the finished catalyst.

2. Catalyst according to claim 1,
**characterised in that**
the aluminium oxide exhibits a specific surface area of between 180 and 400 m²/g.

3. Process for the production of the catalyst according to claim 1 comprising the following process steps
a) impregnation of the moulding of aluminium oxide with an aqueous, alkaline solution of caesium tungstate,
b) drying of the resultant catalyst precursor at room temperature for two or more hours and
c) subsequent calcination for a period of 2 to 10 hours at temperatures of 300 to 500°C.

4. Use of the catalyst according to claims 1 to 2 for the synthesis of alkyl mercaptans from alkanols and hydrogen sulfide.

5. Use of the catalyst according to claims 1 to 2 for the synthesis of methyl mercaptan from methanol and hydrogen sulfide.

## Revendications

1. Catalyseur en un corps moulé en oxyde d'aluminium pour la synthèse de méthylmercaptan, qui contient un tungstate alcalin comme promoteur, **caractérisé en ce qu'**il s'agit, pour le promoteur, de tungstate de césium, qui est appliqué en une quantité de plus de 15 à 40 % en poids par rapport au poids total du catalyseur fini.

2. Catalyseur selon la revendication 1, **caractérisé en ce que** l'oxyde d'aluminium présente une surface spécifique entre 180 et 400 m²/g.

3. Procédé pour la préparation du catalyseur selon la revendication 1 comprenant les étapes de procédé suivantes
a) imprégnation des corps moulés en oxyde d'aluminium avec une solution aqueuse, alcaline de tungstate de césium,
b) séchage des précurseurs de catalyseur obtenus à température ambiante pendant plusieurs heures et
c) calcination finale pendant une durée de 2 à 10 heures à des températures de 300 à 500°C.

4. Utilisation du catalyseur selon les revendications 1 à 2 pour la synthèse d'alkylmercaptans à partir d'alcanols et d'acide sulfhydrique.

5. Utilisation du catalyseur selon les revendications 1 à 2 pour la synthèse de méthylmercaptan à partir de méthanol et d'acide sulfhydrique.
